(19) Europäisches Patentamt
European Patent Office
Office européen des brevets

(11) Veröffentlichungsnummer: **0 000 199**
**B1**

(12) # EUROPÄISCHE PATENTSCHRIFT

(45) Veröffentlichungstag der Patentschrift: **03.03.82**

(51) Int. Cl.³: **C 07 H 15/22,**
A 61 K 31/70

(21) Anmeldenummer: **78100255.5**

(22) Anmeldetag: **28.06.78**

(54) **4-O-Aminoglycosyl-1,3-diaminocyclitole und Verfahren zu deren Herstellung.**

(30) Priorität: **05.07.77 DE 2730372**

(43) Veröffentlichungstag der Anmeldung:
**10.01.79 Patentblatt 79/1**

(45) Bekanntmachung des Hinweises auf die Patenterteilung:
**03.03.82 Patentblatt 82/9**

(84) Benannte Vertragsstaaten:
**BE CH DE FR GB NL**

(56) Entgegenhaltungen:
**DE - A - 2 414 416**
**FR - A - 2 232 326**

**BULL. CHEM. SOC. JAP. 1973, 46 (11) Seite 3507—10**

**JOURNAL OF THE CHEMICAL SOCIETY (C) 1971, Seite 3126—3129**

**JOURNAL OF THE CHEMICAL SOCIETY PERKIN I, 1976, Seite 1088—1097**

(73) Patentinhaber: **BAYER AG**
**Zentralbereich Patente, Marken und Lizenzen**
**D-5090 Leverkusen 1, Bayerwerk (DE)**

(72) Erfinder: **Paulsen, Hans, Prof. Dr.**
**Hinsbeker Berg 11**
**D-2000 Hamburg 65 (DE)**
Erfinder: **Jansen, Rolf**
**Geesthachter Kehre 7**
**D-2000 Hamburg 73 (DE)**
Erfinder: **Stadler, Peter, Dr.**
**Ohligser Strasse 85**
**D-5657 Haan (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 199 B1

# 4-O-Aminoglycosyl-1,3-diaminocyclitole und Verfahren zu deren Herstellung

Die Erfindung betrifft neue Pseudodisaccharide und Verfahren zu ihrer Herstellung. Die neuen Verbindungen können als Arzneimittel sowie als Zwischenprodukte bei der Synthese von Pseudotrisacchariden Verwendung finden.

Die neuen Pseudodisaccharide lassen sich durch die nachstehende Formel (I) wiedergeben

$$X—O—Y \qquad (I)$$

in der

Y für einen Rest

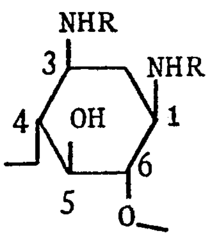 , 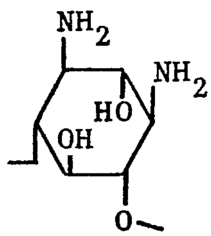 , 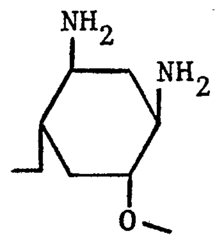 , 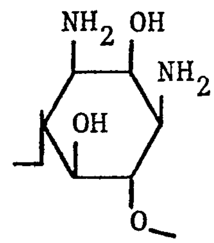

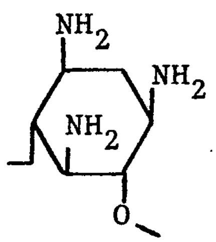 oder 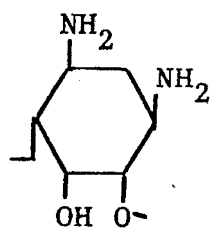

steht und

(a) falls Y einen Rest

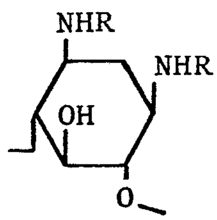

darstellt, X für einen Rest

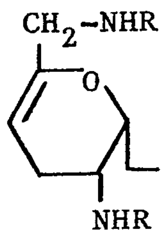

steht,

b) falls Y einen anderen Rest darstellt, X für den Rest

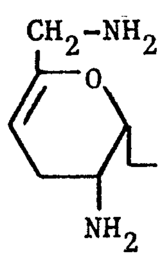

steht, oder

c) für den Fall, daß Y für den Rest

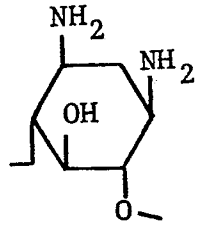

steht, X auch die Reste

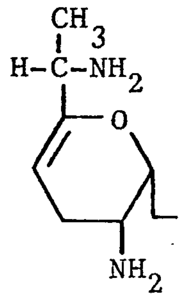

oder

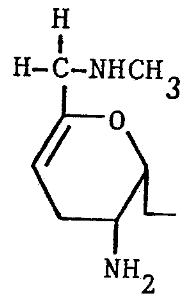

bezeichnen kann, wobei X jeweils (1→4)-glykosidisch mit Y verbunden ist, und wobei die Reste R gleich sind und für Wasserstoff oder

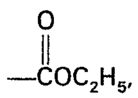

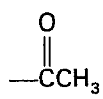

oder

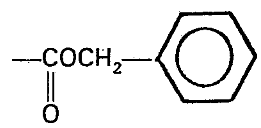

stehen sowie deren physiologisch unbedenkliche Säureadditionssalze, wobei Säureadditionssalze der Verbindungen

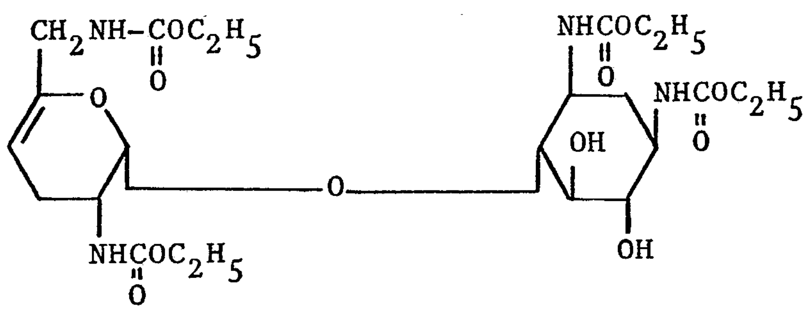

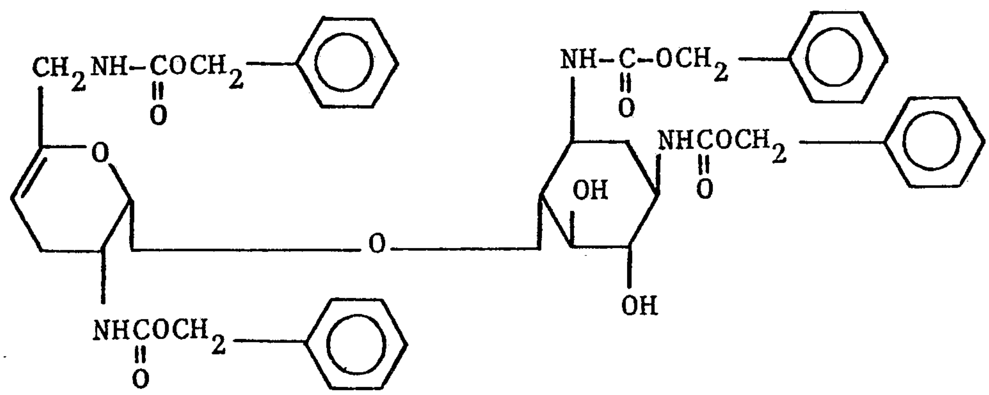

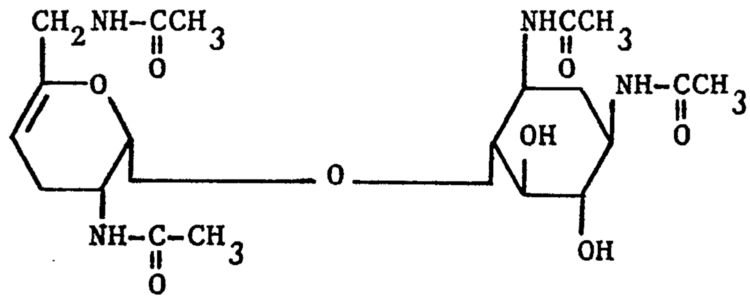

ausgenommen sind.

Ein besonders wertvolles Pseudodisaccharid ist die Verbindung der Formel (II)

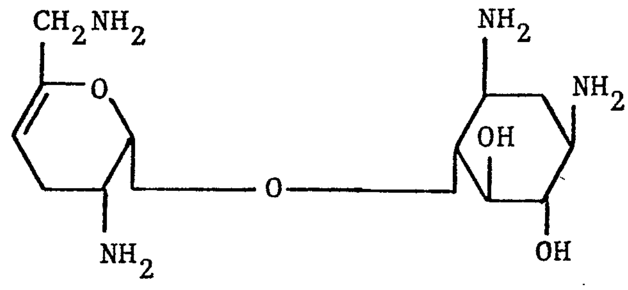

(II)

Als Säureadditionssalze seien beispielhaft jene der Pseudodisaccharide mit Chlorwasserstoff-, Schwefel-, Phosphor-, Salpeter-, Bromwasserstoff-, Benzolsulfon-, Ameisen-, Essig-, Propion-, Malein-, Ascorbin- oder Zitronensäure genannt.

Die Erfindung betrifft weiterhin ein Verfahren zur Darstellung von Verbindungen der Formel (I), welches dadurch gekennzeichnet ist, daß man Verbindungen der Formel

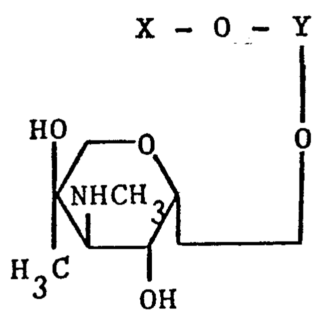

(III)

in der X und Y die oben genannte Bedeutung haben, wobei der Rest Y in 4-Stellung glykosidisch mit X und in 6-Stellung glykosidisch mit dem Garosaminylrest verknüpft ist,
gegebenenfalls in Form N-geschützter Derivate, durch Behandlung mit einem Oxydationsmittel bei Temperaturen von —20 bis 100°C und einem pH-Wert zwischen 3 und 12 spaltet, gegebenenfalls vorhandene Schutzgruppen entfernt und die Produkte in freier Form oder bei der Anwesenheit von Säure in Form ihrer Säureadditionssalze isoliert.

Die Ausgangsverbindungen der Formel (III) sind literatur-bekannte Pseudotrisaccharide wie die Antibiotika Verdamicin, Sisomicin, G 52, Mutamicin 1, Mutamicin 2, Mutamicin 4, Mutamicin 5 und Mutamicin 6 (siehe DT—OS 2 437 160).

Kennzeichnend für die erfindungsgemäßen Pseudodisaccharide ist, daß sich die Doppelbindung in Vinylstellung zum Ringsauerstoff befindet. Aus der Literatur (Bull. Chem. Soc. Jap. 1973, 46, Seite 3508) ist bereits ein strukturell ähnliches Pseudodisaccharid bekannt, bei welchem allerdings die Doppelbindung in Allylstellung zum Ringsauerstoff steht. Aufgrund des völlig andersartigen Verhaltens von Allyl- und Vinylverbindungen waren die erfindungsgemäßen Pseudodisaccharide hierdurch nicht nahegelegt. Darüberhinaus wird die bekannte Verbindung erzeugt durch synthetische Einführung der Doppelbindung zwischen den C-Atomen 3 und 4 in einem Neaminderivat, d. h. einem gesättigten Pseudodisaccharid. Bei Kenntnis der Lehre der genannten Literaturstelle bestand für den Fachmann weder die Veranlassung noch die Möglichkeit, ausgehend von einem derartigen Neaminderivat eine Doppelbindung in der 4,5-Stellung einzuführen. Das erfindungsgemäße Verfahren weist somit erstmals einen Weg, auf welchem 4,5-ungesättigte Pseudodisaccharide überhaupt hergestellt werden können. Die erfindungsgemäßen Pseudodisaccharide weisen als solche bereits gute antibiotische Eigenschaften auf und sind hierin bekannten Verbindungen (beispielsweise dem Neamin der FR—A

2 232 326) überlegen. Sie können jedoch, wie unten näher ausgeführt wird, auch als Ausgangsmaterial für die Herstellung wertvoller neuer und bekannter Pseudotrisaccharide dienen.

In einigen Fällen empfiehlt es sich, bei der Darstellung der erfindungsgemäßen Pseudodisaccharide von selektiv N-blockierten Derivaten von Verbindungen der Formel (III) auszugehen.

Die Art der Blockierung sollte so beschaffen sein, daß alle im Molekül vorhandenen Amino- oder Methylaminogruppen, mit Ausnahme der im abzuspaltenden Garosaminylrest vorhandenen Methylaminogruppe, blockiert sind.

Bei den solcherart geschützten Verbindungen handelt es sich demnach um 1,2′, 3,6′-Tetra-N-blockierte bzw. im Falle von Mutamicin 5 um 1,2′, 3,5,6′-Penta-N-blockierte Derivate der Aminotrisaccharide gemäß Formel (III).

Als Aminoschutzgruppen können alle unter den Oxidationsbedingungen des oben geschilderten Verfahrens stabilen, im Bereich der Aminozucker- und der Peptidchemie üblichen Schutzgruppen verwendet werden. Solche Schutzgruppen und die Verfahren zu ihrer Herstellung sind bekannt (s. z.B. Houben-Weyl, Methoden der organischen Chemie, Bd. XV, 1, Georg Thieme Verlag, Stuttgart, 1974).

Bevorzugt werden in die Oxidationsreaktion solche N-geschützten Derivate der Aminotrisaccharide gemäß Formel (III) eingesetzt, die Acylschutzgruppen vom Typ

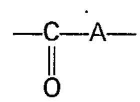

wobei die Bedeutung von A den Formeln (1) und (2) zu entnehmen ist—

$$-(CH_2)_n-B \qquad (1)$$

oder

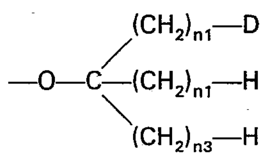

(2)

enthalten, wobei in den genannten Schutzgruppenresten

B und D unabhängig voneinander für Wasserstoff, Phenyl oder substituiertes Phenyl stehen und
$n$, $n_1$, $n_2$, $n_3$ unabhängig voneinander eine Zahl von 0 bis 5 bezeichnen.

Zur Herstellung dieser selektiv geschützten Aminotrisaccharide setzt man nach einem neuen Verfahren (veröffentlichte europäische Patentanmeldung Nr. 0000057) ein Aminotrisaccharid gemäß Formel (III) mit einer Verbindung der Formel (IV)

$$A-CO-G' \qquad (IV)$$

in der

A für einen Rest der Formeln (1) oder (2) steht und
G′ Halogen oder eine bei Acylierungsreaktionen gebräuchliche Abgangsgruppe, vorzugsweise eine aktivierenden Ester, oder eine Gruppe —O—CO—A mit der obigen Bedeutung von A bezeichnet, in einem inerten Lösungsmittel gegebenenfalls unter Zusatz von Wasser bei Temperaturen zwischen etwa −80°C und etwa +50°C in Gegenwart einer Base um und arbeitet das Reaktionsprodukt in üblicher Weise auf.

Zur Spaltung der Verbindungen gemäß Formel (III) bzw. deren N-blockierten Derivaten können übliche Oxidationsmittel verwendet werden.

Beispiele für derartige Oxidationsmittel, die im erfindungsgemäß einzuhaltenden pH-Bereich zwischen 3 und 12 eingesetzt werden können, sind Schwermetallsalze, Peroxide, Halogene, Salze von Halogensauerstoffsäuren, Stickstoffoxide sowie molekularer Sauerstoff. Bevorzugte Oxidationsmittel sind Permanganate, Manganate, Mangandioxid, Chromtrioxid, Bichromate, Chromate, Alkylchromate, Chromylchlorid, Selendioxid, Kobalt (III)-Salze, Cer (IV)-Salze, Kaliumhexacyanoferrat (III), Kupferoxid, Bleioxid, Quecksilberoxid, Gemische von Wasserstoffperoxid mit Eisen (II)-Salzen, Eisen (III)-Salze, Selendioxid, Osmiumtetraoxid, Vanadate, Bleitetraacetat, Chlor, Brom, Jod, Hypochlorite, Chlorate, Hypobromite, Bromate, Perjodate, Distickstoffmonoxid, Stickstoffdioxid und Luft. Bei Verwendung von molekularem Sauerstoff werden vorzugsweise Edelmetalle wie Platin, Palladium, Rhodium, Ruthenium oder Rhenium sowie Nickel als Katalysatoren verwendet.

Besonders bevorzugte Oxidationsmittel sind Mangandioxid, Natriumperjodat, Kaliumhexacyanoferrat (III) und Kaliumpermanganat.

Die Spaltungsreaktion wird vorzugsweise in Gegenwart eines unter den Reaktionsbedingungen inerten Verdünnungsmittels, bevorzugt eines solchen, in dem sich die Reaktionsteilnehmer lösen, durchgeführt. Geeignete Verdünnungsmittel der genannten Art sind Methanol, Ethanol, i-Propanol, Tetrahydrofuran, Dimethylformamid, Dioxan, Pyridin und Ethylenglykoldimethylether, Aceton und Essigsäure sowie Wasser oder Gemische von Wasser mit den genannten organischen Lösungsmitteln.

Die erfindungsgemäße Umsetzung wird je nach Art des verwendeten Oxidationsmittels bei einem pH-Wert von 3 bis 12 durchgeführt. Die Einstellung des pH-Wertes kann durch Zusatz einer entsprechenden Säure oder Base erreicht werden. Dabei sind solche Säuren oder Basen zu verwenden, die die Ausgangsverbindungen oder die Endprodukte nicht zersetzen und keine Aktivitätsverringerung der Oxidationsmittel hervorrufen. Vielmehr ist es wünschenswert, daß sie die Aktivität der Oxidationsmittel erhöhen. Als anorganische Säuren können beispielsweise Salzsäure oder Schwefelsäure und als organische Säuren beispielsweise Essigsäure oder Ameisensäure verwendet werden. Beispiele für entsprechende Basen sind Ammoniumhydroxid, Alkalimetallhydroxide, Erdalkalimetallhydroxide, Alkalimetallcarbonate, Erdalkalimetallcarbonate, Alkalimetallalkoholate und Alkali- und Erdalkalimetallsalze von Carbonsäuren.

Die erfindungsgemäße Umsetzung wird bei Temperaturen von etwa –20 bis etwa 100°C, vorzugsweise von etwa 0 bis etwa 70°C. Die Reaktionsdauer beträgt eine halbe Stunde bis 50 Stunden. Im allgemeinen wird die Umsetzung bei Normaldruck ausgeführt.

Die Abspaltung der nach der Reaktion im Molekül gegebenenfalls noch vorhandenen Schutzgruppen gelingt im bekannter Weise durch alkalische oder saure Hydrolyse, selektive Hydrogenolyse oder Verdrängungsreaktionen. Setzt man zur Spaltung Verbindungen ein, die Acylschutzgruppen vom Typ (1) oder (2) enthalten, so können diese vorzugsweise mit wäßrigen Laugen von Alkali- oder Erdalkalihydroxiden gespalten werden.

Verwendet man z.B. 1,2', 3,6'-Tetra-N-ethyloxycarbonylsicomicin und Natriumperjodat als Ausgangsstoffe, so kann der Reaktionsverlauf durch das folgende Schema wiedergegeben werden:

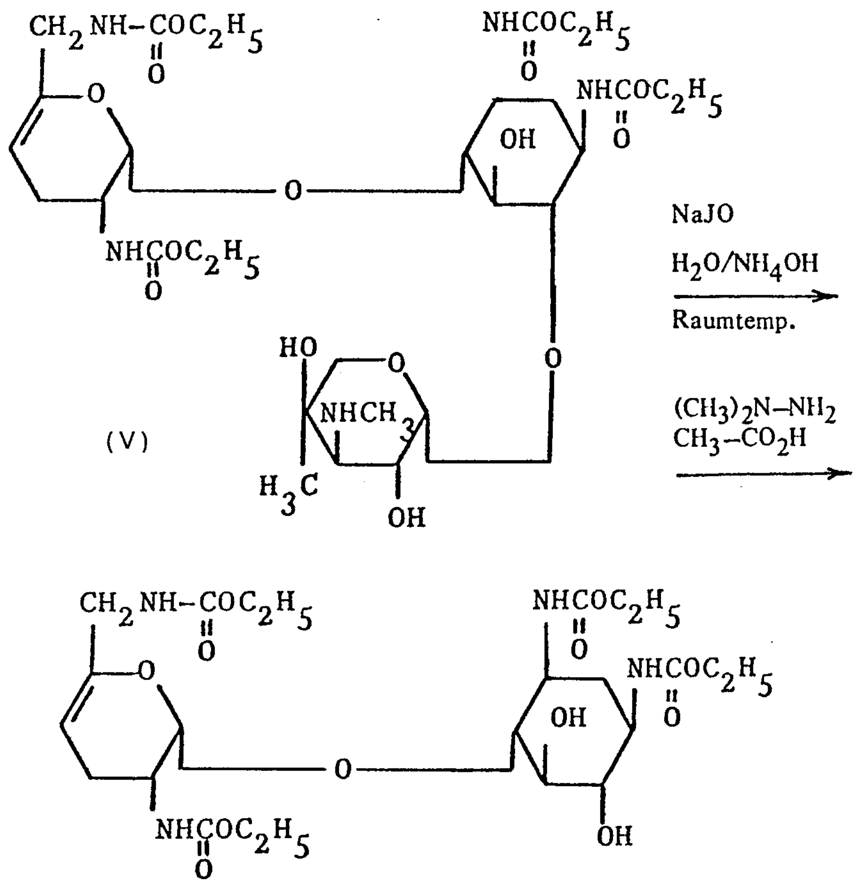


(V)

(VI)

Die erfindungsgemäß verwendbare Ausgangsverbindung V ist gemäß dem in der veröffentlichten europäischen Patentanmeldung 0000057 beschriebenen Verfahren durch Umsetzung von Sisomicin mit Pyrokohlensäurediethylester in wäßrigem Alkohol zugänglich. Die oxidative Abspaltung von (V) kann im Prinzip mit den vorstehend genannten Oxidationsmitteln durchgeführt werden; zu bevorzugen ist im vorliegenden Falle jedoch die Verwendung von Natriumperjodat, das in Form einer wäßrigen Lösung eingesetzt wird. Als Lösungsmittel für die Spaltungsreaktion eignet sich im vorliegenden Falle

insbesondere Methanol oder ein Methanol-Wasser-Gemisch. Man arbeitet vorteilhafterweise mit einem Zusatz von 5—10% konzentriertem Ammoniumhydroxid. Die bevorzugte Reaktionstemperatur beträgt −5° bis +5°C. Die Weiterverarbeitung erfolgt durch Eindampfen des Reaktionsgemisches in Vakuum, Extrahieren des Rückstandes mit Ethanol und Entionisieren der Extrakte mit basischem Ionenaustauscherharz ($OH^{\ominus}$Form).

Das so erhaltene Reaktionsprodukt kann direkt verwendet oder gewünschtenfalls durch Chromatographie gereinigt oder dem "Barry-Abbau", d.h. der Umsetzung mit N,N-Dimethylhydrazin in Essigsäure [vgl. P. S. O'Colla in Methods in Carbohydrate Chemistry *5*, 382—392 (1965)] zugeführt werden.

Die Tetra-N-ethyloxycarbonylverbindung der Formel (VI) wird nach der Aufarbeitung als kristallines Produkt in hoher Ausbeute gewonnen. Die Abspaltung der Schutzgruppen in wäßriger alkalischer Lösung liefert das bisher unbekannte Disaccharid der Formel (VII)

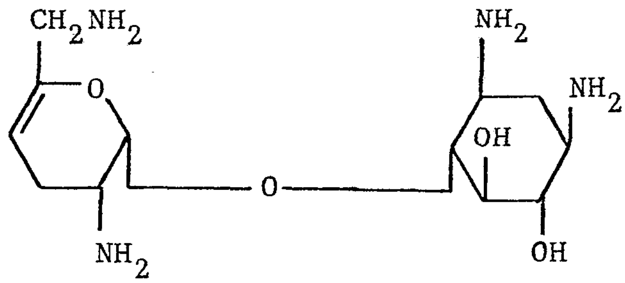

(VII)

Die erfindungsgemäßen Verbindungen sind antimikrobielle Mittel mit einem breiten Wirkungsspektrum und besonderer Wirksamkeit gegen gram-negative Bakterien. Diese Eigenschaften ermöglichen ihre Verwendung als Arzneimittel bei der Bekämpfung von bakteriellen Erkrankungen bei Mensch und Tier. Sie sind gut zur Prophylaxe und Chemotherapie von lokalen und systemischen Infektionen in der Human- und Tiermedizin die durch gram-negative Bakterien, z.B. E. coli, Proteus, Klebsiella und Pseudomonas verursacht werden, geeignet.

Des weiteren handelt es sich bei den erfindungsgemäßen Disacchariden um äußerst wertvolle Zwischenprodukte für die Synthese von Pseudotrisacchariden, die als wertvolle Antibiotika Verwendung finden können.

So eröffnet sich durch die Verwendung der erfindungsgemäßen Zwischenprodukte ein neuer, vorteilhafter Weg zur Herstellung der bekannten Antibiotika 66—40 B und 66—40 D, die bisher lediglich bei der fermentativen Darstellung von Sisomicin zu jeweils etwa 2% gebildet wurden (DT—OS 24 37 160).

Hierbei kann man das bei der oxidativen Spaltung von z.B. Tetra-N-acylsisomicinen gebildete Tetra-N-acyldisaccharid, das noch zwei freie Hydroxylgruppen besitzt im Zuge einer Glykosidierungsreaktion nach den in der Kohlenhydratchemie bekannten Methoden mit einem reaktiven Monosaccharidderivat wie z.B. einem entsprechend geschütztem Glykosylhalogenid der 3-Desoxy-3-methylamino-L-arabinopyranose umsetzen, wobei die glykosidische Verknüpfung erwartungsgemäß an de Hydroxylgruppe an C-6 eintritt. [S. Umezawa, Advan. Carbohydr. Chem. *30*, 111 (1974)].

Die anschließende Entblockierung der Glykosidierungsprodukte nach bekannten Methoden liefert das gewünschte Pseudotrisaccharid.

Im Falle der Verwendung von partiell blockierten Aminotrisacchariden für die Oxidationsreaktion erhält man N-geschützte Pseudodisaccharide (siehe z.B. Formel VI), die sich nach den bekannten Glykosidierungsreaktionen in semisynthetische Aminotrisaccharide überführen lassen.

## Beispiel 1

4-O-(2,6-Diamino-2,3,4,6-tetradesoxy-$\alpha$-D-glycero-hex-4-enopyranosyl)-2-desoxystreptamin

2 g (4,47 mMol) Sisomicin in 20 ml Wasser werden bei 0°C langsam mit einer Lösung von 2,88 g (13,4 mMol) $NaJO_4$ in 40 ml Wasser unter Rühren versetzt. Nach 2 Stunden wird im Hochvakuum zur Trockne eingedampft. Der Rückstand wird mit Ethanol aufgerührt und nach 15 Minuten filtriert.

Das Filtrat wird zum Sirup eingeengt, in 40 ml Wasser aufgenommen, mit 2,4 ml = 1,9 g (32 mMol) N,N-Dimethylhydrazin versetzt, mit Essigsäure auf pH 6 eingestellt und bei 40°C über Nacht gelagert. Die braungelbe Lösung wird verdünnt auf sauren Ionenaustauscher (R) Amberlite IRC 50, $H^{+}$Form gegeben. Aus dem gründlich neutral gewaschenen Ionenaustauscher wird das Produkt mit 2 N Ammoniak eluiert. Das Eluat wird am Hochvakuum zur Trockne eingedampft. Man erhält einen amorphen, farblosen Feststoff.

## Beispiel 2

4-O-(2,6-Di-benzyloxycarbonylamino-2,3,4,6-tetradesoxy-$\alpha$-D-glycero-hex-4-enopyranosyl)-1,3-di-N-benzyloxycarbonyl-2-desoxystreptamin

Das nach Beispiel 1 erhaltene Produkt wird in 7 ml Methanol/Wasser 5:2 mit 0,85 ml Carbobenzoxychlorid und 0,3 g $Na_2CO_3$ über Nacht gerührt. Das Gemisch wird mit Wasser verdünnt, bis alle Salze gelöst sind und filtriert. Der Rückstand wird mit Wasser, Methanol und Petroläther gewaschen, in möglichst wenig $CHCl_3$ aufgerührt und erneut filtriert. Der weiße Rückstand wird mit wenig $CHCl_3$ gewaschen und aus Pyridin mit Methanol umgefällt.

Schmp. = 244—246,5°C $[\alpha]_D^{28} = +61,5°$ (c = 1,0 Pyridin)

| | | | |
|---|---|---|---|
| Analyse für $C_{44}H_{48}N_4O_{12}$: | ber. C 64,06 | H 5,87 | N 6,79 |
| | gef. C 63,69 | H 5,88 | N 6,79 |

Beispiel 3

4-O-(2,6-Di-ethyloxycarbonylamino-2,3,4,6-tetradesoxy-$\alpha$-D-glycero-hex-4-enopyranosyl)-1,3-di-N-ethyloxycarbonyl-2-desoxystreptamin

Zu einer Lösung von 950 mg (1,3 mMol) Tetra-N-ethyloxycarbonyl-sisomicin in 15 ml Methanol und 1 ml konz. Ammoniumhydroxid werden unter Rühren bei 0°C langsam 0,83 g (3,9 mMol) $NaJO_4$ in 10 ml Wasser getropft. Nach Entfernung der Kühlung wird eine Stunde bei Raumtemperatur gerührt und anschließend am Hochvakuum zur Trockne eingedampft. Der Rückstand wird in 30 ml Methanol aufgerührt, mit 30 ml Ethanol versetzt, 15 Minuten gerührt und filtriert. Der Rückstand wird mit Ethanol gewaschen. Das Filtrat wird mit stark basischen Ionenaustauscher (Amberlite ® IRA 400) behandelt und zum Sirup eingeengt. Der Sirup wird mit 40 ml Methanol aufgenommen, mit 0,89 ml = 0,7 g (11,6 mM) N,N-Dimethylhydrazin versetzt, mit Essigsäure auf pH 6,5 eingestellt und bei 40°C über Nacht gelagert. Die braungelbe Lösung wird mit Wasser verdünnt, mit $Na_2CO_3$ neutralisiert und im Hochvakuum zur Trockne eingedampft. Der Rückstand wird mit $CHCl_3$ aufgerührt und mit Wasser ausgeschüttelt. Die wäßrige Phase wird abgetrennt. Die organische Phase wird über $MgSO_4$ getrocknet und eingeengt. Der Rückstand wird in weniger Methanol aufgenommen, mit Ether verdünnt und nach Zugabe von Petrolether in der Tiefkühlung gelagert. Es ergeben sich 370 mg, in einer zweiten Abscheidung 150 mg Tetra-N-carboethoxy-sisomicin als schwach gelbes Pulver. Ausbeute 530 mg (71%). Zur Charakterisierung wird aus Methanol/Ether mit Petrolether umgefällt. (Dünnschichtchromatographie: Toluol, Triethylamin, Methanol 10:3:1)

$[\alpha]_{-D}^{23}$ +107° (c = 1,0 $CH_3OH$) Fp 213,5—215,0°C

| | | | |
|---|---|---|---|
| Analyse für $C_{24}H_{44}N_4O_{15}$: | ber. C 49,65 | H 7,64 | N 9,65 |
| | gef. C 49,68 | H 7,03 | N 9,60 |

Beispiel 4

4-O-(2,6-Di-acetamido-2,3,4,6-tetradesoxy-$\alpha$-D-glycero-hex-4-enopyranosyl)-1,3-di-N-acetyl-2-desoxystreptamin

1,9 g 1,2',3,6'-Tetra-N-acetylsisomicin in 20 ml Wasser werden bei 60°C zu einer Lösung von 3 g Kaliumhexacyanoferrat-(III) und 900 mg Kaliumhydroxid in 20 ml Wasser getropft. Anschließend tropft man eine Lösung von 9 g Kaliumhexacyanoferrat-(III) und 1 g Kaliumhydroxid in 90 ml Wasser innerhalb 1,5 Stunden dazu. Nach beendeter Zugabe wird mit 250 ml Aceton versetzt, vom ausgefallenen anorganischen Material abfiltriert und das Filtrat im Vakuum von Lösungsmittel befreit. Der so erhaltene Rückstand wird mit 10 ml einer aus gleichen Teilen bestehenden Mischung aus Methylenchlorid und Methanol extrahiert, die Extrakte filtriert und in Vakuum eingedampft. Man erhält so 1,1 g der Titelverbindung, die zur Reinigung aus Ethanol/Ether kristallisiert wird.

$[\alpha]_D^{23} = +170°$ (c = 1,0 $CH_3OH$) Fp 238,5—242,0°C

| | | | |
|---|---|---|---|
| Analyse für $C_{20}H_{32}N_4O_8$: | ber. C 52,61 | H 7,06 | N 12,27 |
| | gef. C 51,92 | H 7,19 | N 11,87 |

Beispiel 5

4-O-(2,6-Diamino-2,3,4,6-tetradesoxy-$\alpha$-D-glycero-hex-4-enopyranosyl)-2-desoxystreptamin aus 4-O-(2,6-Di-acetamido-2,3,4,6-tetradesoxy-$\alpha$-D-glycero-hex-4-enopyranoxyl)-1,3-di-N-acetyl-2-desoxystreptamin

410 mg 4-O-(2,6-Di-acetamido-2,3,4,6-tetradesoxy-$\alpha$-D-glycero-hex-4-enopyranosyl)-1,3-di-N-acetyl-2-desoxystreptamin und 5 g $Ba(OH)_2 \times 8H_2O$ werden in 5 ml Wasser 6 Stunden bei einer Ölbadtemperatur von 110—120°C unter Rühren erhitzt. Dann wird mit Wasser verdünnt, durch Zugabe von festem Kohlendioxid neutralisiert und durch eine Glasfritte filtriert. Der Rückstand wird mit Wasser gewaschen. Das Filtrat wird auf ca. 40 ml eingeengt, mit 2 N $H_2SO_4$ auf pH 6 eingestellt, filtriert und zur Trockne eingedampft. Der Rückstand wird in wenig Methanol/Wasser aufgenommen, filtriert und getrocknet. Ausbeute 421 mg (97%) amorpher Feststoff als Sulfat.

**Patentansprüche**

1. Verbindungen der Formel (I)

X—O—Y—H (I)

in der
Y für einen Rest

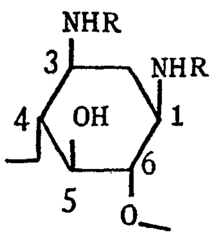
,
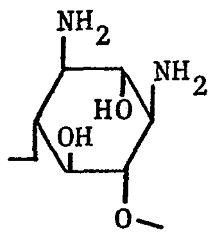
,
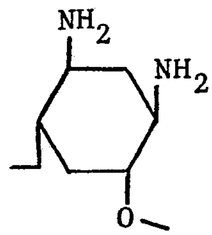
,
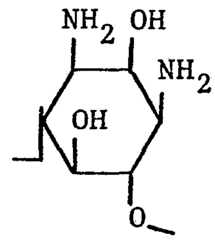

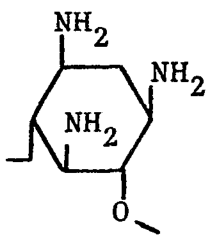
oder
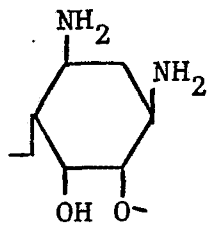

steht und
a) falls Y einen Rest

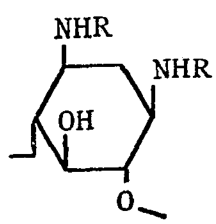

darstellt, X für einen Rest

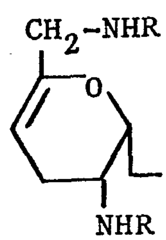

steht,
b) falls Y einen anderen Rest darstellt, X für den Rest

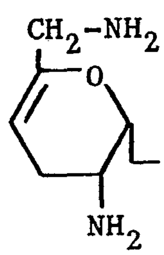

steht, oder

c) für den Fall, daß Y für den Rest

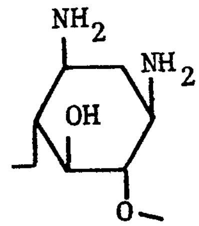

steht, X auch die Reste

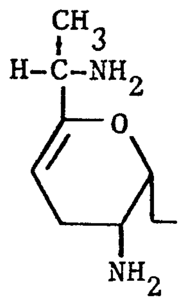

oder

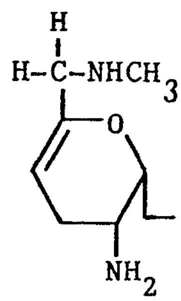

bezeichnen kann, wobei X jeweils (1→4)-glykosidisch mit Y verbunden ist, und wobei die Reste R gleich sind und für Wasserstoff oder

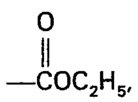

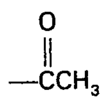

oder

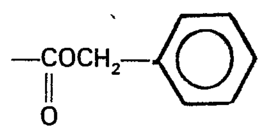

stehen sowie deren physiologisch unbedenkliche Säureadditionssalze, wobei Säureadditionssalze der Verbindungen

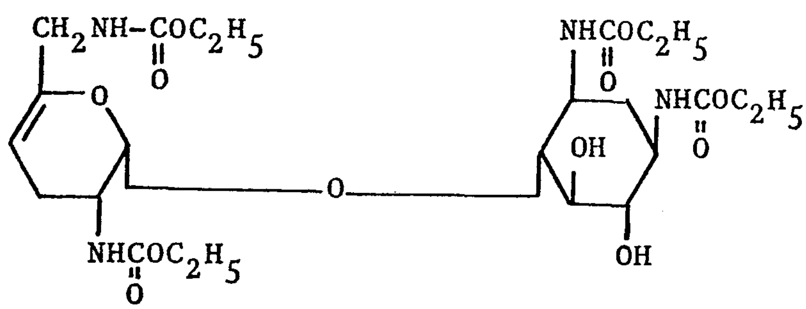

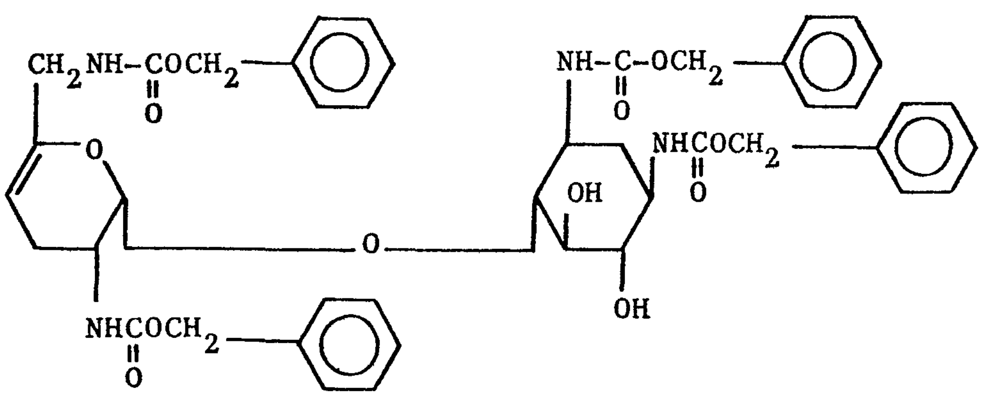

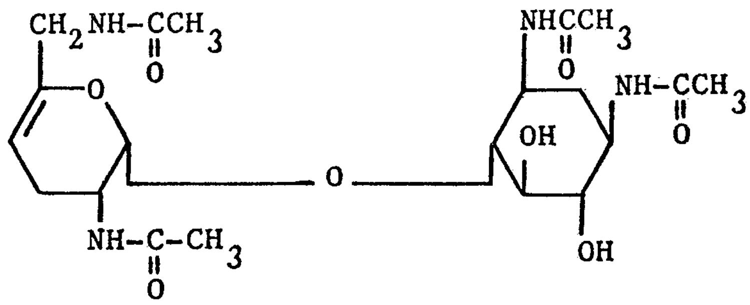

ausgenommen sind.

2. Verbindungen der Formel

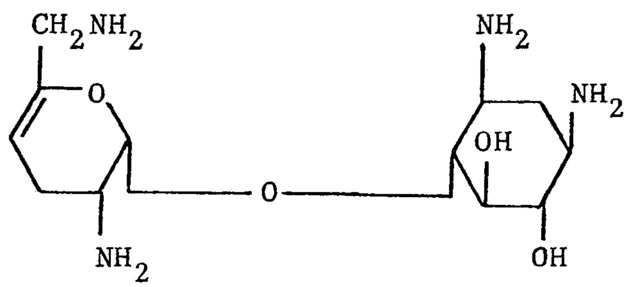

sowie deren physiologisch unbedenkliche Säureadditionssalze.

3. Verbindung der Formel

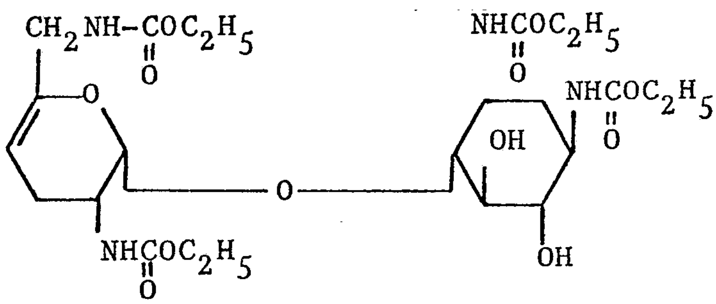

4. Verbindungen der Formel

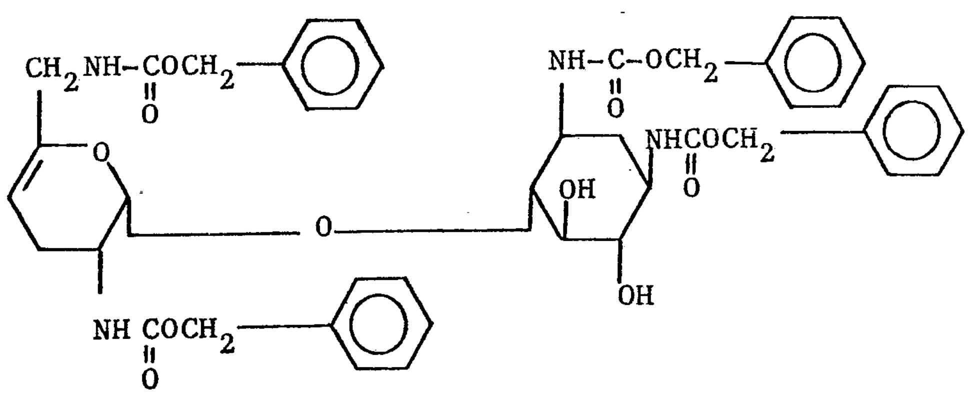

5. Verbindung der Formel

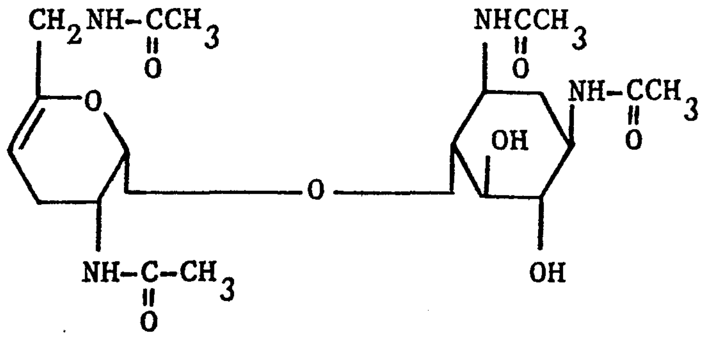

6. Verfahren zur Herstellung von Verbindungen der Formel (I) gemäß Anspruch 1, dadurch gekennzeichnet, daß man Verbindungen der Formel

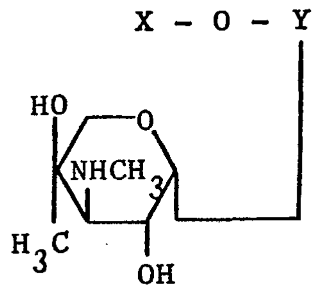

(III)

in der

X und Y die in Anspruch 1 genannte Bedeutung haben, wobei der Rest Y in 4-Stellung glykosidisch mit X und in 6-Stellung glykosidisch mit dem Garosaminylrest verknüpft ist, gegebenenfalls in Form N-geschützter Derivate, durch Behandlung mit einem Oxydationsmittel bei Temperaturen von −20 bis 100°C und einem pH-Wert zwischen 3 und 12 spaltet, gegebenenfalls vorhandene Schutzgruppen entfernt und die Produkte in freier Form oder bei der Anwesenheit von Säure in Form ihrer Säureadditionssalze isoliert.

7. Verfahren nach Anspruch 6, dadurch gekennzeichnet, daß man die Oxidationsreaktion in Gegenwart eines unter den Reaktionsbedingungen inerten Verdünnungsmittels ausführt.

8. Verfahren nach Anspruch 6 oder 7, dadurch gekennzeichnet, daß man als Oxidationsmittel Schwermetallsalze, Peroxide, Halogene, Salze von Halogensauerstoffsäuren, Stickstoffoxide oder molekularen Sauerstoff einsetzt.

9. Verfahren nach Anspruch 6 bis 8, dadurch gekennzeichnet, daß man als Oxidationsmittel Mangandioxid, Natriumperjodat, Kaliumhexacyanoferrat (III) oder Kaliumpermanganat einsetzt.

**Revendications**

1. Composés répondant à la formule (I):

X—O—Y—H (I)

dans laquelle Y représente un radical

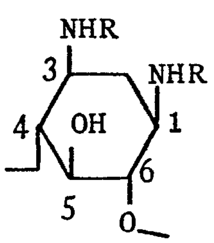 , 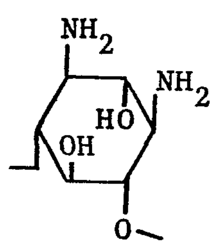 , 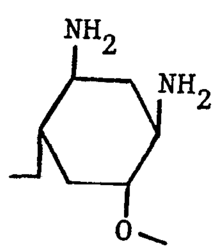 , 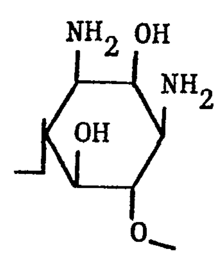

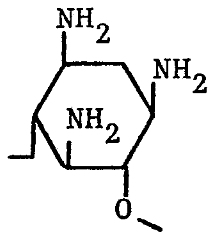 ou 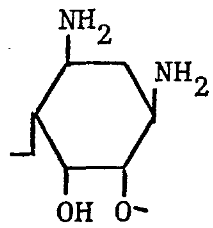 et

a) si Y représente un radical

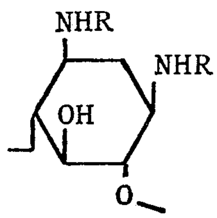

X est un radical

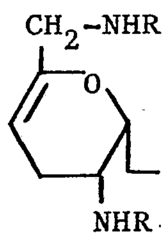

b) si Y représente un autre radical, X représente le radical

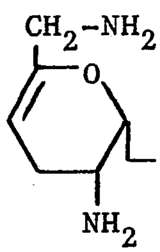 ou

c) si Y représente le radical

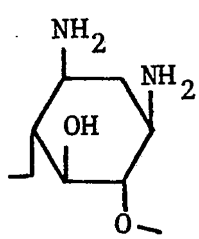

X peut également représenter les radicaux

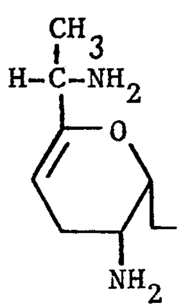 ou 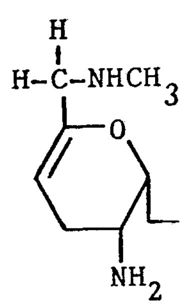

X étant chaque fois relié à Y par une liaison (1→4)-glycosidique et les radicaux R étant identiques et représentant chacun un atome d'hydrogène ou

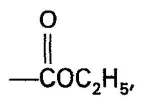, 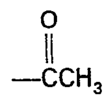 ou 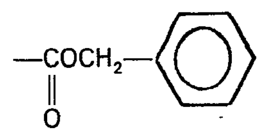

de même que leurs sels d'addition d'acide physiologiquement acceptables, à l'exception des sels d'addition d'acide des composés

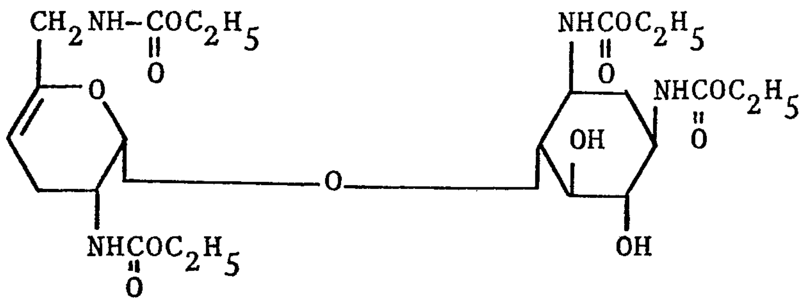

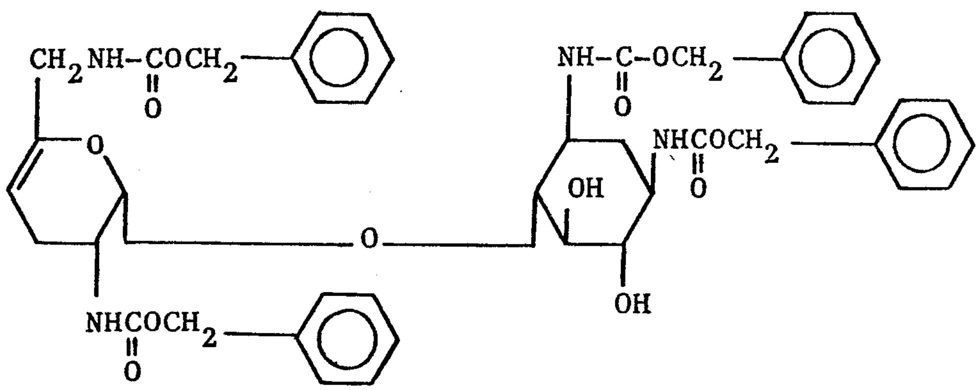

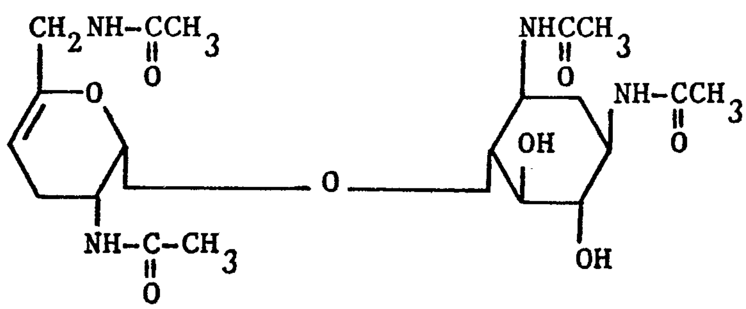

2. Composés de formule:

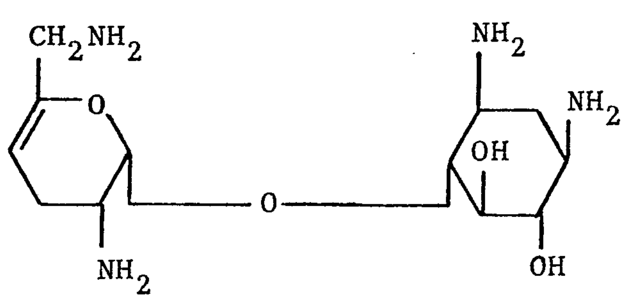

ainsi que leurs sels d'addition d'acide physiologiquement acceptables.

3. Composé de formule:

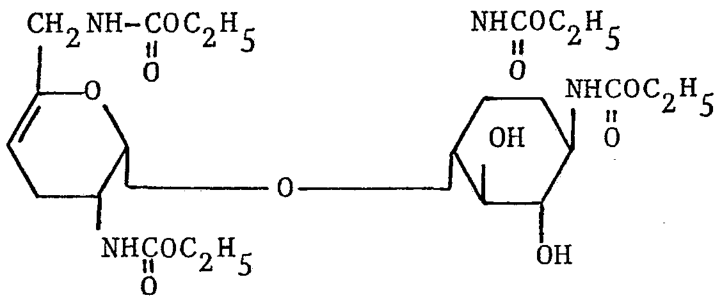

4. Composés de formule:

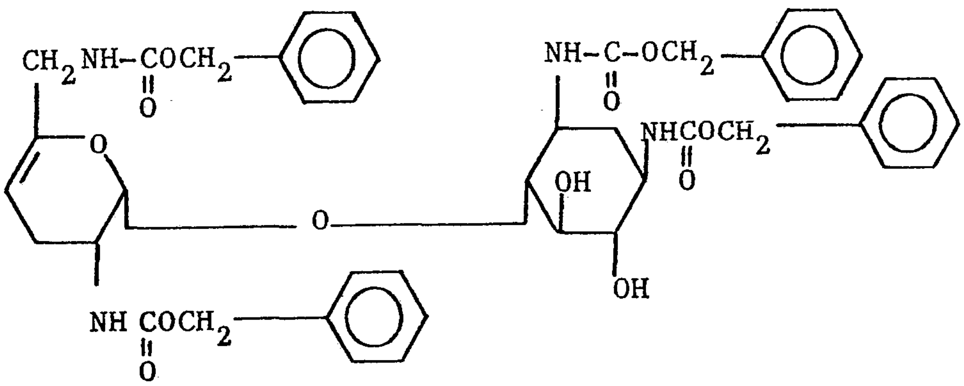

5. Composé de formule:

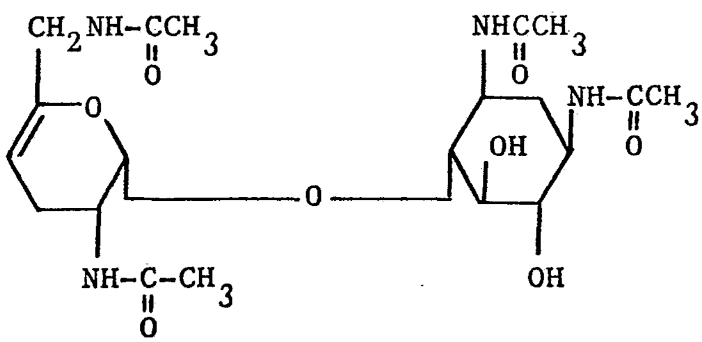

6. Procédé de préparation de composés de formule (I) suivant la revendication 1, caractérisé en ce qu'on scinde des composés de formule:

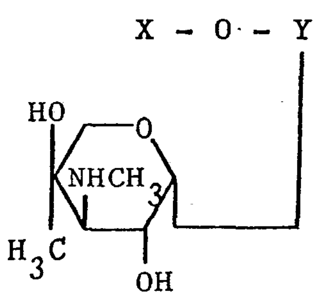

(III)

dans laquelle

X et Y ont les significations indiquées dans la revendication 1, le radical Y étant relié au radical X en position 4 par une liaison glycosidique et, en position 6, au radical garosaminyle également par une liaison glycosidique,

éventuellement sous forme de dérivés N-protégés, par traitement avec un agent d'oxydation à des températures de −20 à 100°C et à un pH compris entre 3 et 12, on élimine les groupes protecteurs éventuellement présents et on isole les produits sous forme libre, ou, en présence d'un acide, sous forme de leurs sels d'addition d'acide.

7. Procédé suivant la revendication 6, caractérisé en ce qu'on effectue la réaction d'oxydation en présence d'un diluant inerte dans les conditions réactionnelles.

8. Procédé suivant la revendication 6 ou 7, caractérisé en ce que, comme agents d'oxydation, on utilise des sels de métaux lourds, des peroxydes, des halogènes, des sels d'hydracides halogénés, des oxydes d'azote ou l'oxygène moléculaire.

9. Procédé suivant les revendications 6 à 8, caractérisé en ce que, comme agent d'oxydation, on utilise le dioxyde de manganèse, le periodate de sodium, l'hexacyanoferrate(III) de potassium ou le permanganate de potassium.

**Claims**

1. Compounds of the formula (I)

$$X—O—Y—H \qquad (I)$$

in which

Y represents a radical

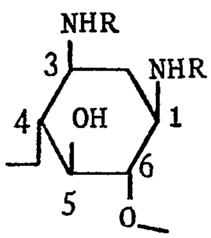 ,

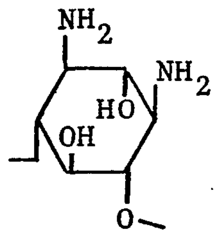 ,

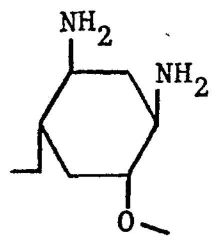 ,

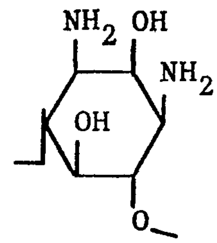

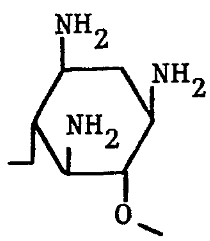 or 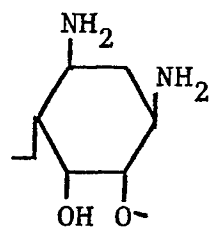

and

a) if Y represents a radical

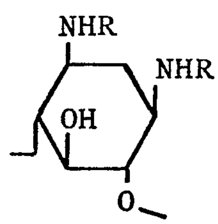

X represents a radical

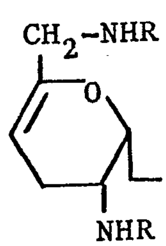

b) if Y represents another radical, X represents the radical

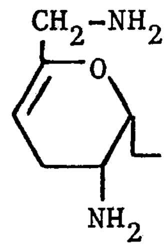

c) in the case where Y represents the radical

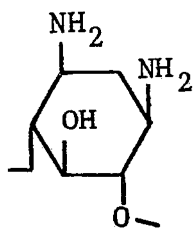

X can also denote the radicals

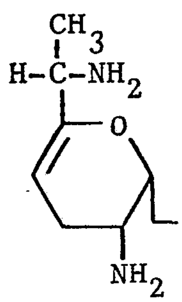

or

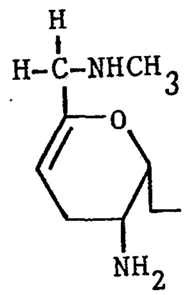

wherein the bond between X and Y is a (1→4)-glycosidic bond in each case, and wherein the radicals R are identical and represent hydrogen or

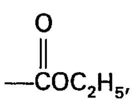

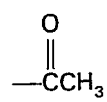

or

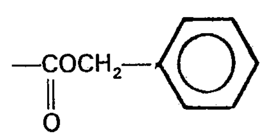

and physiologically acceptable acid addition salts thereof, with the exception of acid addition salts of the compounds

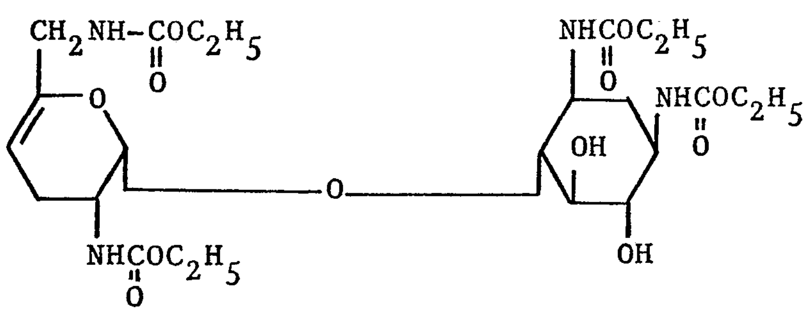

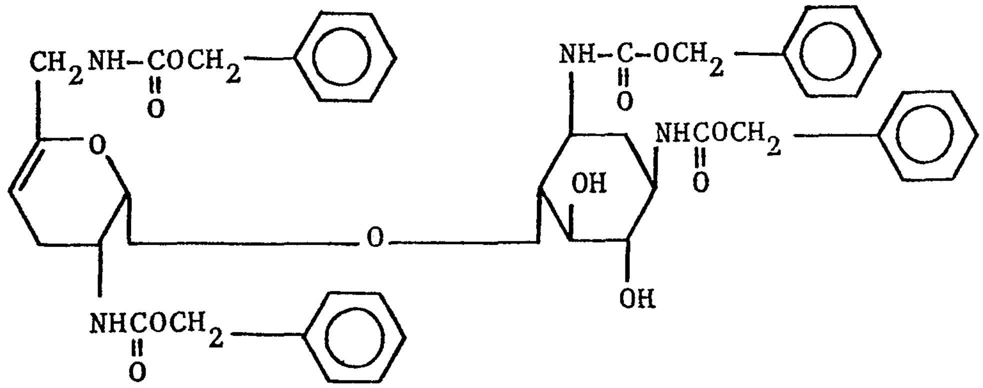

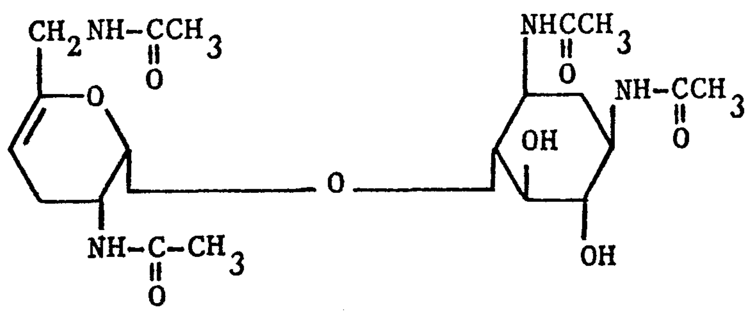

2. Compounds of the formula

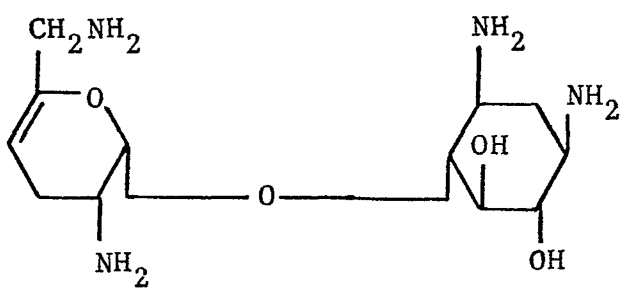

and physiologically acceptable acid addition salts thereof.

3. Compound of the formula

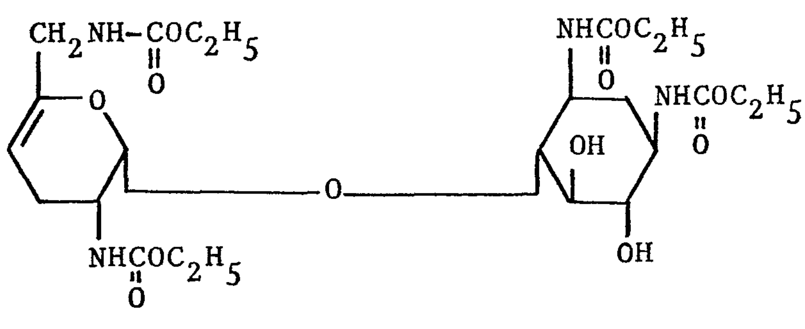

4. Compounds of the formula

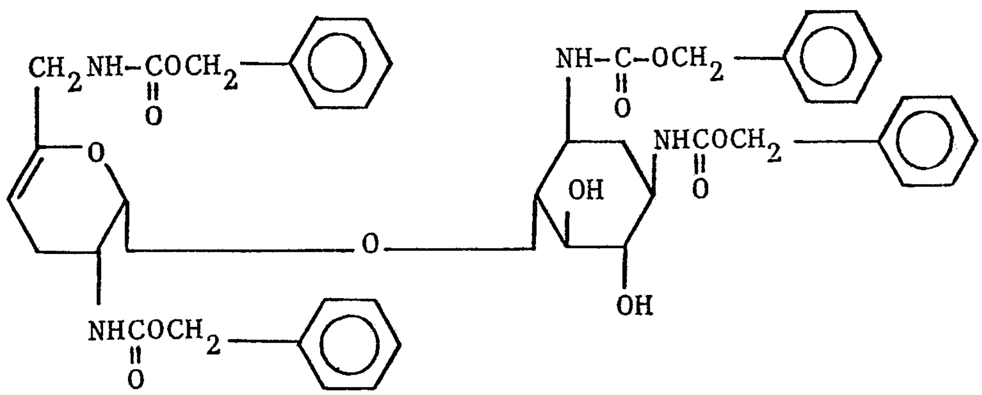

5. Compound of the formula

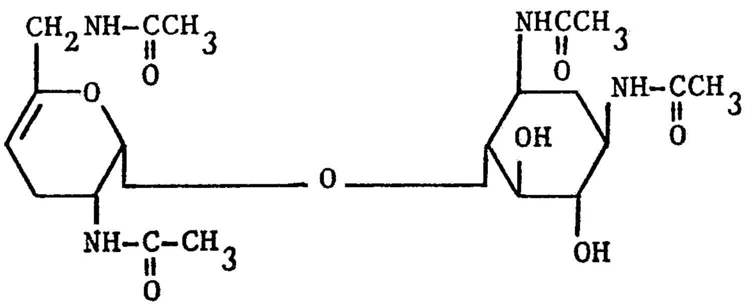

6. Process for the preparation of compounds of the formula (I) according to Claim 1, characterised in that compounds of the formula

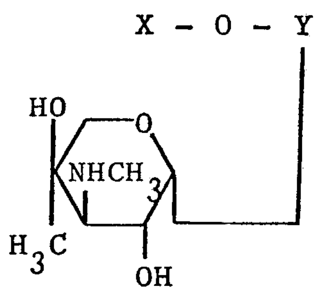

(III)

in which

X and Y have the meaning given in Claim 1, wherein the radical Y is linked glycosidically with X in the 4-position and glycosidically with the garosaminyl radical in the 6-position, optionally in the form of N-protected derivatives, are cleaved by treatment with an oxidising agent at temperatures from —20 to 100°C and at a pH value between 3 and 12, any protective groups which may be present are removed and the products are isolated in their free form or, if an acid is present, in the form of their acid addition salts.

7. Process according to Claim 6, characterised in that the oxidation reaction is carried out in the presence of a diluent which is inert under the reaction conditions.

8. Process according to Claim 6 or 7, characterised in that heavy metal salts, peroxides, halogens, salts of hydrogen halide acids, nitrogen oxides or molecular oxygen are used as oxidising agent.

9. Process according to Claim 6 to 8, characterised in that manganese dioxide, sodium periodate, potassium hexacyanoferrate-III or potassium permanganate are used as oxidising agent.